# EUROPEAN PATENT SPECIFICATION

(11) **EP 3 056 159 B1**
(45) Date of publication and mention of the grant of the patent: **01.05.2019**
(21) Application number: 14860655.1
(22) Date of filing: 26.12.2014
(51) Int. Cl.: A61B 18/12, A61B 1/00, A61B 17/34

(54) **MEDICAL DEVICE**
MEDIZINISCHES INSTRUMENT
INSTRUMENT MÉDICAL

(30) Priority: 11.11.2013 JP 2013233253
(43) Date of publication of application: 17.08.2016
(73) Proprietor: Olympus Corporation, Tokyo 192-8507 (JP)
(72) Inventor: TSURUTA, Shoei, Hachioji-shi, Tokyo 192-8507 (JP)
(74) Representative: Gunzelmann, Rainer
(86) International application number: PCT/JP2014/084651
(87) International publication number: WO 2015/068862

(56) References cited:
- EP-A1- 0 888 749
- EP-A1- 2 147 650
- EP-A1- 2 308 405
- WO-A1-2014/054399
- DE-A1- 3 708 801
- JP-A- H08 112 245
- JP-A- H11 128 242
- JP-A- S61 118 713
- JP-A- 2002 017 663
- JP-U- S6 371 003
- US-A1- 2002 128 644
- US-A1- 2011 152 859

## Description

### Technical Field

The present invention relates to a medical device to receive or transmit power wirelessly.

### Background Art

A trocar is integrally combined with an inner needle having a sharp puncture needle at the forward end, and the inner needle in such a state is punctured through a body wall of a patient so as to be inserted into the abdominal cavity. After being inserted into the abdominal cavity, the inner needle is removed so as to leave the trocar at the body wall, and then the trocar is used as a guide tube for a treatment tool that is for treatment in the abdominal cavity.

Some treatment tools inserted into a body of the subject via an insertion hole of a trocar are connected to a cable to receive power required for the treatment. Such a cable hinders the manipulation by an operator during operation and degrades the operability.

To solve this problem, JP H11-128242 A discloses a system such that a treatment tool inserted in a trocar wirelessly receives power via AC magnetic field generated from a power-transmission coil of the trocar.

In the case of a treatment tool including a manipulation wire to transmit the manipulation by an operator mechanically, however, AC magnetic field generated at the power-transmission coil is applied to the manipulation wire as well, meaning that high-frequency induction current may flow through the manipulation wire. The manipulation wire is inserted to the manipulation part manipulated by the operator, and so the high-frequency current may flow through the manipulation part internally. The induction current flowing through the manipulation wire may adversely affect the power transmission/reception efficiency.

Meanwhile in the case of a flexible endoscope equipped with a power-transmission coil as well, when AC magnetic field is generated to transmit power to a treatment tool inserted into a channel wirelessly, induction current may flow through a manipulation wire of a manipulation part with which an operator manipulates a curved part.

A medical device has been demanded, which is configured so that no induction current flows through a manipulation wire of a manipulation part manipulated by an operator.

Document US 2011/152859 A1 discloses a surgical instrument comprising a first electrode, a second electrode, and a retractable sheath. At least one of the electrodes can comprise an insulative jacket extending along the length thereof which can comprise a tissue stop for limiting the progression of the electrode into tissue.

### Citation List

### Patent Document

Patent Document 1: JP HII-128242 A

### Summary of the Invention

### Problems to be solved by the Invention

An embodiment of the present invention aims to provide a medical device which is configured so that no induction current flows through a manipulation wire of a manipulation part manipulated by an operator.

### Means for solving the Problems

The present invention concerns a medical device according to claim 1.
A medical device that is one embodiment of the present invention includes: a manipulation part that is manipulated by an operator; an insertion part that is elongated from the manipulation part and is inserted into a body of a subject; and a manipulation wire that passes through the insertion part to transmit manipulation at the manipulation part to a forward-end side of the insertion part. The manipulation wire includes a first wire having one end connected to the forward-end side, to which AC magnetic field or AC electrical field is applied, and a second wire having one end connected to the manipulation part and the other end that is connected to the first wire but is insulated electrically from the first wire.

### Advantageous Effect of the Invention

An embodiment of the present invention can provide a medical device which is configured so that no induction current flows through a manipulation wire of a manipulation part manipulated by an operator.

### Brief Description of the Drawings

FIG. 1 is a partial cross-sectional view to describe the operating state of a treatment tool of a first example.
FIG. 2 is an enlarged partial cross-sectional view to describe the operating state of a treatment tool of the first example.
FIG. 3 is a cross-sectional view to describe a connecting part of a manipulation wire of the treatment tool in the first example.
FIG. 4 is an exploded cross-sectional view to describe a connecting part of a manipulation wire of the treatment tool that is modification example 1 of the first example.
FIG. 5A is a cross-sectional view to describe a manipulation wire of a treatment tool that is modification example 2 of the first example.
FIG. 5B is a cross-sectional view taken along the line VB-VB of FIG. 5A to describe the treatment tool that is modification example 2 of the first example.
FIG. 6 is a cross-sectional view to describe a manipulation wire of a treatment tool that is modification example 3 of the first example.
FIG. 7A schematically describes a manipulation wire of a treatment tool that is modification example 4 of the first example.
FIG. 7B schematically describes a manipulation wire of a treatment tool that is modification example 5 of the first example.
FIG. 7C schematically describes a manipulation wire of a treatment tool that is modification example 6 of the first example.
FIG. 7D schematically describes a manipulation wire of a treatment tool that is modification example 7 of the first example.
FIG. 8 describes the configurations of a treatment tool of a second example and an endoscope according to the invention.
FIG. 9 is a partial cross-sectional view of the treatment tool and endoscope of the second example.
FIG. 10 is a partial cross-sectional view of the endoscope according to the invention.

### Mode for carrying out the Invention

### <First Example>

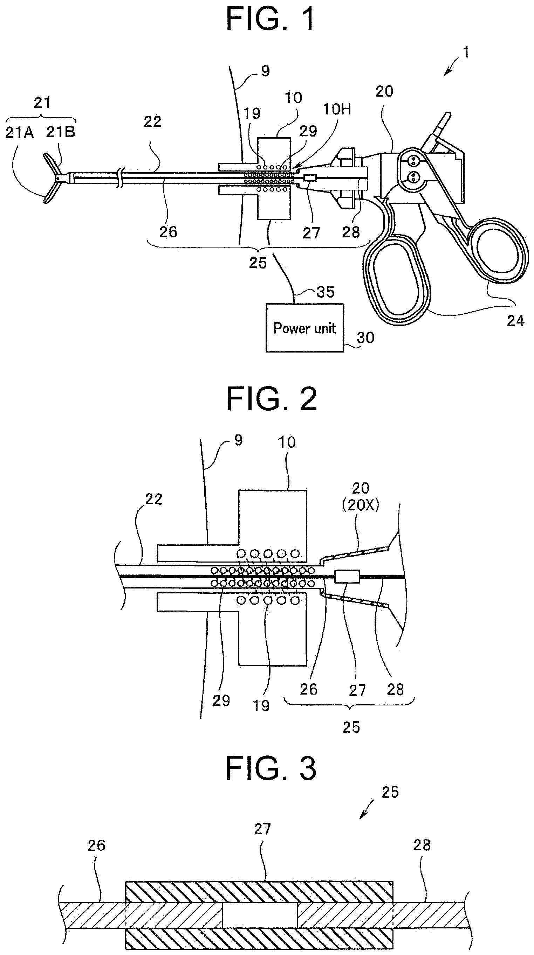

Referring to FIGs. 1 to 3, the following describes a treatment tool 20 that is a medical device of a first example. As illustrated in FIG. 1, the treatment tool 20 makes up an operating system 1 with a trocar 10 as an insertion aid and a power unit 30. The treatment tool 20 for surgical operation is inserted into a body of a subject 9 via an insertion hole 10H of the trocar 10 that is punctured through a body wall of the subject 9. Although the operating system 1 allows an endoscope or the like also to be inserted into the body via another trocar, the descriptions thereof are omitted.

The treatment tool 20 that is a high-frequency treatment tool includes: a manipulation part 24; an insertion part 22 that is inserted into a body of the subject 9; a treatment part 21 that is disposed on the forward end side of the insertion part 22; a manipulation wire 25 and a power-reception coil 29. The manipulation wire 25 is made of metal with high rigidity, e.g., stainless steel (SUS) or nickel titanium (Ni-Ti) alloy, through which the insertion part 22 is inserted, and is configured to transmit the manipulation at the manipulation part 24 to the treatment part 21.

The treatment part 21 for treatment in the body of the subject 9 includes a pair of jaws 21A and 22B that can open/close. For instance, when the operator grasps the manipulation part 24, the jaws 21A and 22B are closed, and when the operator opens the manipulation part 24, the jaws 21A and 22B are open.

While pinching tissue to be treated between the jaws 21A and 22B, high-frequency current is applied to the jaws 21A and 22B for treatment, such as incision or stopping of bleeding.

Then power for treatment is transmitted wirelessly to the treatment tool 20 from the trocar 10.

To this end, a solenoid-type power-transmission coil 19 is wound around the insertion hole 10H of the trocar 10 at the outer periphery. When receiving AC power from a power unit 30 via a cable 35, the power-transmission coil 19 generates AC magnetic field.

The power unit 30 outputs high-frequency and large power of 10 W to 100 W, for example. The AC magnetic field generated by the power-transmission coil 19 has a frequency that can be selected appropriately in the range of 100 kHz to 20 MHz, for example, from which a frequency that is allowed for use by law, e.g., 13.56 MHz, is preferably selected.

Then the power-reception coil 29 of the treatment tool 20 is inductively coupled with the power-transmission coil 19 of the trocar 10, and so receives power wirelessly via the AC magnetic field. The power received by the power-reception coil 29 is converted into high-frequency power at a power-reception circuit not illustrated, which is then applied to the jaws 21A and 22B.

In the treatment tool, the manipulation wire 25 is inserted through the power-reception coil 29 internally. This means that AC magnetic field generated by the power-transmission coil 19 of the trocar 10 is applied to the manipulation wire 25 as well. AC magnetic field is applied to the manipulation wire 25 when the manipulation wire is disposed outside of the power-reception coil 29 as well. Induction current therefore flows through the manipulation wire 25.

However, as illustrated in FIG. 2 or the like, the manipulation wire 25 of the treatment tool 20 includes a first wire 26 having one end connected to the treatment part 21 on the forward end side of the insertion part 22, and a second wire 28 having one end connected to the manipulation part 24. Then the first wire 26 and the second wire 28 are connected mechanically, but are insulated electrically.

That is, as illustrated in FIG. 3, for example, the first wire 26 and the second wire 28 are both made of metal, and are connected with a heat-shrinkable tube 27 so that they are not in contact with each other.

The heat-shrinkable tube 27 is a shape-memory plastic tube that shrinks into a pre-memorized shape by heating, which is made of an insulating material, such as silicone resin or fluorine resin.

As illustrated in FIG. 2, the connecting part, i.e., the heat-shrinkable tube 27 is disposed closer to the manipulation part 24 than the power-reception coil 29 when the power-transmission coil 19 and the power-reception coil 29 are electromagnetically coupled. Induction current flows through the first wire 26 due to AC magnetic field generated by the power-transmission coil 19. The induction current, however, does not flow to the second wire 28 from the first wire 26.

That is, the treatment tool 20 is configured so that no induction current flows through the second wire 28 of the manipulation part 24 manipulated by the operator. This can eliminate the risk of heat generated at the second wire 28 or a failure of the intensity of electromagnetic wave to satisfy the standard (ordinance of the Ministry of Internal Affairs and Communications) about specific absorption rate (SAR) due to induction current, for example.

The first wire 26B/the second wire 28 may be used as a signal line or a ground potential line.

High-frequency current may flow through an unexpected path. To cope with this, as illustrated in Fig. 2, the insertion part side of the manipulation part 24 is preferably a member 20X made of a low dielectric loss insulating material. In other words, the treatment tool 20 and the manipulation part 24 are preferably electrically insulated with a member 20X made of a low dielectric loss insulating material, e.g., polystyrene, polyethylene, or fluorine resin having dielectric loss tangent of 0.01 or less. The member 20X may be a connecting member that connects removably with the manipulation part 24 and the insertion part 22.

The above describes a bipolar-type high-frequency treatment tool as the treatment tool 20, which may be various treatment tools having a manipulation wire to manipulate a manipulation part on the forward end side, e.g., a monopolar-type electrosurgical knife, and similar effects can be obtained therefrom.

The above describes the treatment tool 20 that receives power wirelessly via the power-reception coil 29 that is electromagnetically coupled with the power-transmission coil 19 of the trocar 10 as an insertion aid. The treatment tool may be one that receives power wirelessly via a power-reception electrode that is capacitive-coupling with a power-transmission electrode of an insertion aid, and such a configuration of the present invention has the same effects as long as induction current flows through the manipulation wire.

In other words, a medical device includes a manipulation wire that is made up of a first wire having one end connected to the forward end side, to which AC magnetic field or AC electrical field is applied, and a second wire having one end connected to the manipulation part and the other end that is connected to the first wire but is insulated from the first wire, and in such a medical device, no induction current flows through the manipulation wire of the manipulation part manipulated by the operator.

### <Modification Example 1>

The manipulation wire of the treatment tool 20 may include a first wire having one end connected to the treatment part 21 on the forward end side and a second wire having one end connected to the manipulation part 24 and the other end connected to the first wire and being electrically insulated from the first wire.

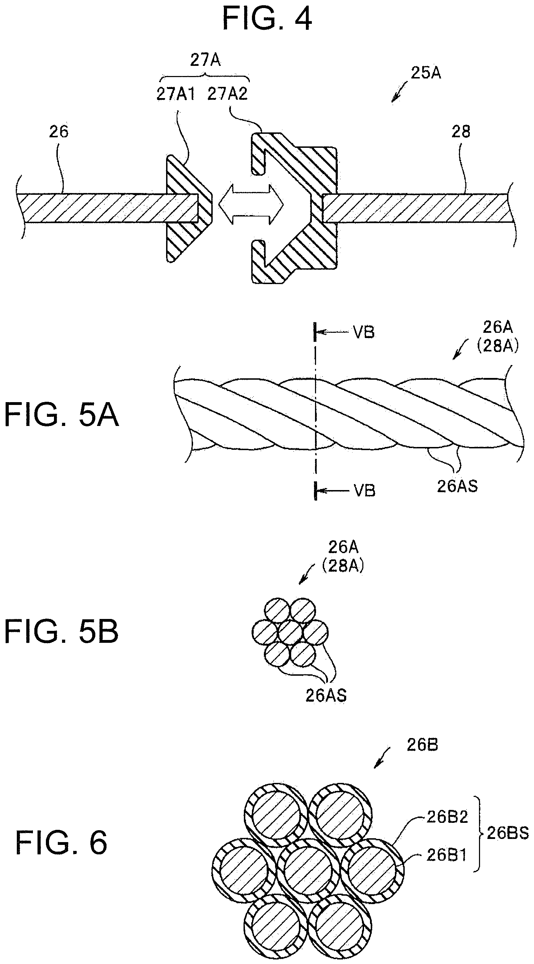

For example, a manipulation wire 25A of the treatment tool as modification example 1 in FIG. 4 includes a first wire 26 made of metal and a second wire 28 made of metal that are connected via a connecting member 27A including a first member 27A1 and a second member 27A2 that fit to each other. That is, the first wire 26 is joined with the first member 27A1, and the second wire 28 is joined with the second member 27A2.

The connecting member 27A may be an insulating body made of fluorine resin such as poly tetra fluoro ethylene (PTFE), or poly ether ether ketone (PEEK).

As long as the first wire 26 and the second wire 28 of the treatment tool are mechanically connected via a connecting member made of an insulating body, such a treatment tool has the same effects as those of the treatment tool 20.

### <Modification Example 2>

As illustrated in FIG. 5A and FIG. 5B, any one of a first wire 26A and a second wire 28A of a treatment tool as modification example 2 is stranded wire including a plurality of metal element wires 26AS made of stainless steel (SUS) or nickel titanium (Ni-Ti) alloy, for example, that are stranded into one.

Stranded wire is not likely to be stretched and has excellent durability against bending as compared with solid wire. That is, the treatment tool of modification example 2 has the advantageous effects of the treatment tool 20 and further has excellent durability, for example.

### <Modification Example 3>

As illustrated in FIG. 6, in the manipulation wire of a treatment tool as modification example 3, at least a first wire 26B is stranded wire 26B including metal element wires 26BS each including metal element wire 26B1 coated with an insulating body 26B2. The second wire also may have the same configuration as that of the first wire 26B.

AC magnetic field is applied to the first wire 26B. Since each metal element wire 26BS is insulated, meaning that eddy current generated has a shorter loop length, loss is small and the amount of heat generated also is small.

When the first wire 26B is used as a signal line, since each metal element wire 26B1 is thin in diameter, increase in electrical resistance due to proximity effect can be suppressed. As a result, the first wire 26B can have good transmission efficiency and the amount of heat generated is small.

Polyurethane is typically used for coating of metal conducting wire. On the other hand, coating of metal element wire used for a treatment tool is preferably made of a material having high heat resistance, such as heat-resistant polyurethane, nylon, polyester, fluorine resin or polyparaxylylene.

### <Modification Example 4>

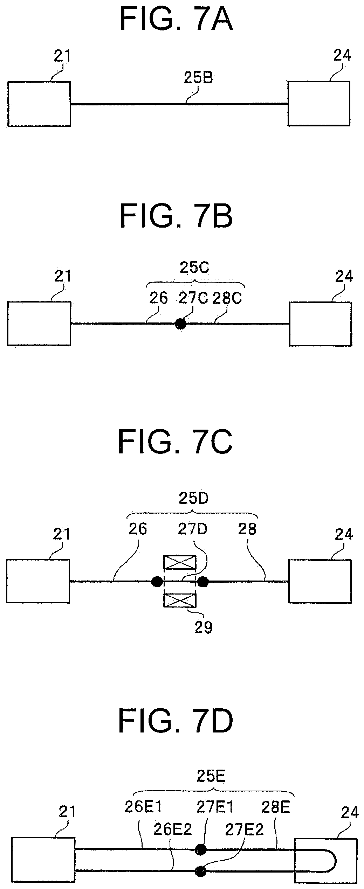

The manipulation wire is preferably made of metal from the viewpoint of elasticity and durability, for example. However, one manipulation wire 25B made of an insulating body illustrated in FIG. 7A may be used in some specifications.

### <Modification Example 5>

As illustrated in FIG. 7B, a manipulation wire 25C may include a first wire 26 made of metal and a second wire 28C made of an insulating body that are connected via a connecting member 27C, and in this configuration also, no induction current flows through the second wire 28C of the manipulation part 24 manipulated by an operator. The connecting member 27C may be an electrically-conductive member.

The insulating wire is preferably made of PEEK with high rigidity. Stranded wire including a plurality of element wires made of an insulating material that are stranded may be used.

### <Modification Example 6>

A manipulation wire 25D illustrated in FIG. 7C includes a connecting member that is an insulating wire 27D made of PEEK, for example. Since intense magnetic field is applied to a part of the wire passing through the power-reception coil 29, i.e., a part passing through the power-transmission coil 19, large induction current easily flows therethrough and eddy current is generated, which may degrade the power transmission/reception efficiency. To cope with this, such a part may be the insulating wire 27D, which can prevent induction current from flowing into the manipulation part side and can prevent the degradation of the power transmission/reception efficiency.

### <Modification Example 7>

A manipulation wire 25E illustrated in FIG. 7D includes two wires 26E1 and 26E2 connected to the treatment part 21, and a wire 28E connected to the wires 26E1 and 26E2 via connecting members 27E1 and 27E2. The wire 28E is mechanically connected to the manipulation part 24 not at an end part but at a center part. In the wire 28E, when the manipulation part 24 rotates and so when the wire 26E1 receives tensile stress to the manipulation part side, for example, the wire 26E2 receives compressive stress to the treatment part side.

Needless to say, in the case of a treatment tool including a plurality of manipulation wires as well, such a treatment tool has the same advantageous effects as those of the treatment tool 20 or the like as long as each manipulation wire is connected via a connecting member made of an insulating body like the manipulation wire 28E.

Although the above describes the manipulation wire 25 including a first wire and a second wire connected, three or more wires may be connected. In that case, a connecting part made of an insulating material may be disposed at least at a part closer to the manipulation part than the position of the power-reception coil.

### <Second Example>

A medical device of the present example is a treatment tool 50 that is inserted into a channel of an endoscope 40. That is, an insertion aid for the treatment tool 50 is the endoscope 40 instead of the trocar 10.

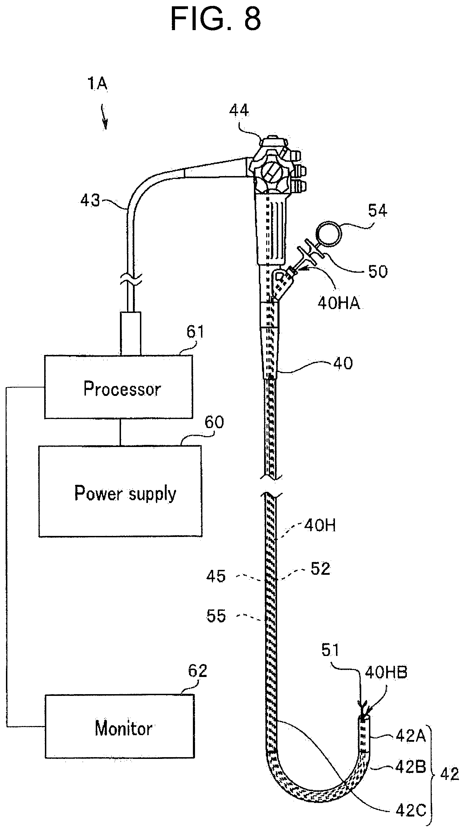

As illustrated in FIG. 8, an operating system 1A includes the treatment tool 50 of the present embodiment, the endoscope 40, a processor 61 that is connected to the endoscope 40 via a universal code 43 and processes a signal, a power supply unit 60 and a monitor 62 to display an image at the endoscope.

The endoscope 40 includes a channel 40H that is an insertion hole into which a long and thin insertion part 42 to be inserted into a body of a subject is inserted. The channel 40H may be a flexible resin tube. The detailed configuration of the endoscope 40 will be described in a first embodiment.

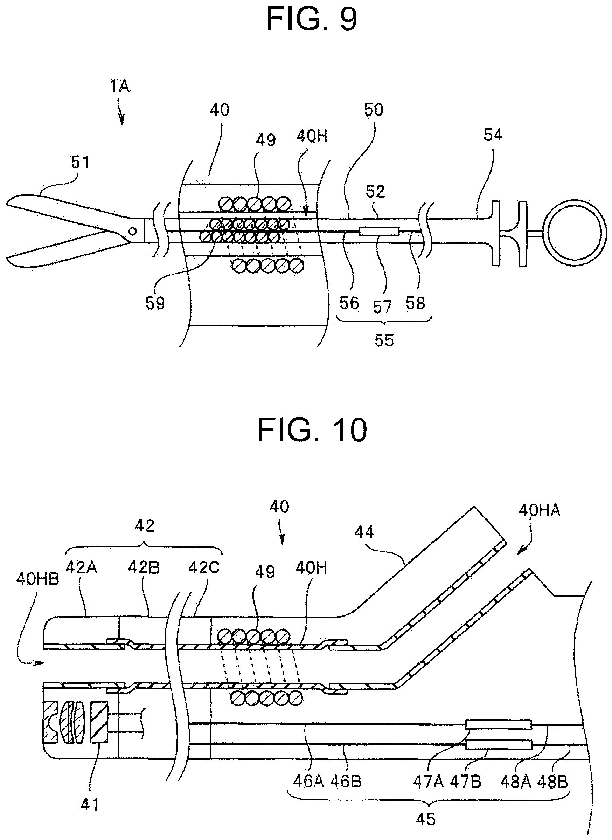

As illustrated in FIG. 9, the treatment tool 50 includes a manipulation part 54 manipulated by an operator, a treatment part 51 on the forward end side, and an insertion part 52 into which a manipulation wire 55 is inserted, the manipulation wire transmitting the manipulation at the manipulation part 54 to the treatment part 51. The treatment tool 50 is inserted from an insertion hole 40HA of the channel 40H in the manipulation part 44 of the endoscope 40, and passes through the insertion part 42 (soft part 42C, curved part 42B, forward-end part 42A), and then the treatment part 51 on the forward end side protrudes from an opening 40HB.

A power-transmission coil 49 wound around the channel 40H of the endoscope 40 generates AC magnetic field. Treatment is then performed at the treatment part 51 with power received by a power-reception coil 59 that is electromagnetically coupled with the power-transmission coil 49.

The manipulation wire 55 of the treatment tool 50 includes a first wire 56 having one end connected to the treatment part 51 on the forward end side of the insertion part 52, a second wire 58 having one end connected to the manipulation part 54, and an insulating connecting member 57. That is, the first wire 56 and the second wire 58 are connected mechanically, but are insulated electrically.

Although the insertion aid for the treatment tool 50 of the present example is the endoscope 40, its basic configuration is the same as that of the treatment tool 20 of the first example, and has the same function. That is, no induction current flows through the manipulation wire 58 of the manipulation part 54 manipulated by an operator.

The configurations of the modification examples of the first example may be used in the treatment tool 50 of the present example as well.

### <First embodiment>

A medical device of the present embodiment includes an endoscope 40 as an insertion aid. That is, an operating system 1A illustrated in FIG. 8 includes the treatment tool 50 of the second example, and the endoscope 40 of the present embodiment.

The endoscope 40 as an insertion aid has a greatly different basic configuration from that of the treatment tool 50 or the like. However, they are common in that AC magnetic field is applied to the manipulation wire.

As already described, the endoscope 40 includes a power-transmission coil 49 that generates AC magnetic field to transmit power to the treatment tool 50 inserted into the channel 40H. Then as illustrated in FIG. 8 and FIG. 10, the endoscope 40 includes a manipulation wire 45 that passes through the insertion part 42, the manipulation wire 45 being to manipulate the curved part 42B that changes the direction of the forward-end part 42A at which an imaging part 41 is disposed.

As illustrated in FIG. 10, the manipulation wire 45 includes a first wire 46A and a second wire 48A that are connected via an insulating connecting member 47A, and a first wire 46B and a second wire 48B that are connected via an insulating connecting member 47B. One ends of the first wire 46A and the other first wire 46B are connected to the curved part 42B on the forward end side. Then the other ends of the second wire 48A and the other second wire 48B are connected to an angle knob of the manipulation part 44. The second wire 48A and the other second wire 48B may be one wire such that the other ends of them on the manipulation part side are connected.

In the endoscope 40, AC magnetic field generated at the power-transmission coil 49 is applied to the manipulation wire 45 as well.

The first wires 46A, 46B and the second wires 48A, 48B, however, are connected mechanically, but are insulated electrically.

Although the endoscope 40 of the present embodiment is an insertion aid, its basic configuration is the same as those of the treatment tools 20 and 50, and has the same function. That is, no induction current flows through the manipulation wire 48A or 48B of the manipulation part 44 manipulated by an operator.

The configurations of the modification examples of the first example may be used in the endoscope 40 of the present embodiment as well.

The present invention is not limited to the above-described embodiments, and can be changed, combined and adapted variously without deviating from the scope of the present invention.

### Description of Reference Numerals

- 1, 1A: operating system
- 10: trocar
- 19: power-transmission coil
- 20: treatment tool
- 21: treatment part
- 22: insertion part
- 24: manipulation part
- 25: manipulation wire
- 26: first wire
- 27: heat-shrinkable tube
- 28: second wire
- 29: power-reception coil
- 30: power unit
- 40: endoscope
- 40H: channel
- 42: insertion part
- 42A: forward-end part
- 42B: curved part
- 44: manipulation part
- 45: manipulation wire
- 49: power-transmission coil
- 50: treatment tool
- 51: treatment part
- 52: insertion part
- 54: manipulation part
- 55: manipulation wire
- 56: first wire
- 57: connecting member
- 58: second wire
- 59: power-reception coil
- 60: power unit

## Claims

1. A medical device (20), comprising:
a manipulation part (24; 54) that is manipulated by an operator;
an insertion part (22; 42) that is elongated from the manipulation part and is inserted into a body of a subject; and
a manipulation wire (25) that passes through the insertion part to transmit manipulation at the manipulation part to a forward-end side of the insertion part, wherein
the manipulation wire includes a first wire (26) having one end connected to the forward-end side, to which AC magnetic field or AC electrical field is applied, and a second wire (28) having one end connected to the manipulation part and the other end that is connected to the first wire but is insulated electrically from the first wire,
**characterized in that** the medical device (20) further comprises
an endoscope (40) including a curved part (42B) on the forward-end side of the insertion part (22; 42), the curved part (42B) being connected to the manipulation wire (25) to change a direction of the forward end in accordance with manipulation at the manipulation part (24; 54);
a channel (40H) that passes through the insertion part (22; 42), into which a treatment tool is inserted; and
a power-transmission part (19; 49) that generates the AC magnetic field or the AC electrical field, and transmits power wirelessly to the treatment tool inserted into the channel (40H).

2. The medical device (20) according to claim 1, wherein the first wire made of metal and the second wire made of metal are connected via a connecting member (20) made of an insulator.

3. The medical device (20) according to claim 2, wherein the connecting member includes wire made of an insulating body.

4. The medical device (20) according to claim 1, wherein at least one of the first wire and the second wire includes stranded wire including a plurality of metal element wires stranded.

5. The medical device (20) according to claim 4, wherein the first wire includes metal element wires each being coated with an insulating body.

6. The medical device (20) according to claim 1, wherein the second wire includes an insulating body.

7. The medical device (20) according to claim 1, wherein the manipulation wire includes an insulating body.

8. The medical device (20) according to any one of claims 1 to 7, wherein the medical device comprises a treatment tool (50) including: a power-reception part (59) disposed at the insertion part of the treatment tool to receive the AC magnetic field or the AC electrical field; and a treatment part (51) that is disposed on the forward-end side to perform treatment with received power.

## Patentansprüche

1. Medizinische Vorrichtung (20), umfassend:
ein Manipulationsteil (24; 54), das von einem Bediener manipuliert wird;
ein Einsetzteil (22; 42), das sich von dem Manipulationsteil länglich erstreckt und in einen Körper eines Subjekts eingesetzt wird; und
ein Manipulationskabel (25), der durch das Einsetzteil hindurchgeht, um die Manipulation an dem Manipulationsteil an eine Seite des Vorderendes des Einsetzteils zu übertragen, wobei
das Manipulationskabel ein erstes Kabel (26) umfasst, das an einem Ende mit der Seite des Vorderendes verbunden ist, an den ein magnetisches Wechselfeld oder ein elektrisches Wechselfeld angelegt wird, und ein zweites Kabel (28), das an einem Ende mit dem Manipulationsteil und an dem anderen Ende mit dem ersten Kabel verbunden, doch elektrisch von dem ersten Kabel isoliert ist,
**dadurch gekennzeichnet, dass** die medizinische Vorrichtung (20) ferner umfasst
ein Endoskop (40), das ein gebogenes Teil (42B) an der Seite des Vorderendes des Einsetzteils (22; 42) umfasst, wobei das gebogene Teil (42B) mit dem Manipulationskabel (25) verbunden ist, um eine Richtung des Vorderendes gemäß der Manipulation an dem Manipulationsteil (24; 54) zu ändern;
einen Kanal (40H), der durch das Einsetzteil (22; 42) hindurchgeht, in den ein Behandlungswerkzeug eingesetzt wird; und
ein Energieübertragungsteil (19; 49), das das magnetische Wechselfeld oder das elektrische Wechselfeld erzeugt und Energie drahtlos an das in den Kanal (40H) eingesetzte Behandlungswerkzeug überträgt.

2. Medizinische Vorrichtung (20) nach Anspruch 1, wobei das erste aus Metall bestehende Kabel und das zweite aus Metall bestehende Kabel über ein aus einem Isolator bestehendes Verbindungselement (20) verbunden sind.

3. Medizinische Vorrichtung (20) nach Anspruch 2, wobei das Verbindungselement ein aus einem Isolierkörper bestehendes Kabel umfasst.

4. Medizinische Vorrichtung (20) nach Anspruch 1, wobei mindestens eines des ersten Kabels und des zweiten Kabels Litzendraht umfasst, der eine Vielzahl von verdrillten Metallelementdrähten umfasst.

5. Medizinische Vorrichtung (20) nach Anspruch 4, wobei das erste Kabel Metallelementdrähte umfasst, die jeweils mit einem Isolierkörper beschichtet sind.

6. Medizinische Vorrichtung (20) nach Anspruch 1, wobei das zweite Kabel einen Isolierkörper umfasst.

7. Medizinische Vorrichtung (20) nach Anspruch 1, wobei das Manipulationskabel einen Isolierkörper umfasst.

8. Medizinische Vorrichtung (20) nach einem der Ansprüche 1 bis 7, wobei die medizinische Vorrichtung ein Behandlungswerkzeug (50) umfasst, umfassend:
ein Energieempfangsteil (59), das an dem Einsetzteil des Behandlungswerkzeugs angeordnet ist, um das magnetische Wechselfeld oder das elektrische Wechselfeld zu empfangen; und
ein Behandlungsteil (51), das auf der Seite des Vorderendes angeordnet ist, um eine Behandlung mit der empfangenen Energie durchzuführen.

## Revendications

1. Dispositif médical (20), comprenant :
une partie de manipulation (24 ; 54) qui est manipulée par un opérateur ;
une partie d'insertion (22 ; 42) qui est allongée à partir de la partie de manipulation et est insérée dans le corps d'un sujet ; et
un fil de manipulation (25) qui passe à travers la partie d'insertion pour transmettre une manipulation au niveau de la partie de manipulation à un côté extrémité avant de la partie d'insertion,
le fil de manipulation comprenant un premier fil (26) ayant une extrémité reliée au côté extrémité avant, auquel un champ magnétique CA ou un champ électrique CA est appliqué, et un second fil (28) ayant une extrémité reliée à la partie de manipulation et l'autre extrémité qui est reliée au premier fil mais est isolée électriquement du premier fil,
**caractérisé par le fait que** le dispositif médical (20) comprend en outre
un endoscope (40) comprenant une partie incurvée (42B) sur le côté extrémité avant de la partie d'insertion (22 ; 42), la partie incurvée (42B) étant reliée au fil de manipulation (25) pour changer une direction de l'extrémité avant en fonction d'une manipulation au niveau de la partie de manipulation (24 ; 54) ;
un canal (40H) qui passe à travers la partie d'insertion (22 ; 42), dans lequel un outil de traitement est inséré ; et
une partie de transmission d'énergie (19 ; 49) qui génère le champ magnétique CA ou le champ électrique CA, et transmet de l'énergie de manière sans fil à l'outil de traitement inséré dans le canal (40H).

2. Dispositif médical (20) selon la revendication 1, dans lequel le premier fil fait de métal et le second fil fait de métal sont reliés par l'intermédiaire d'un élément de liaison (20) fait d'un isolant.

3. Dispositif médical (20) selon la revendication 2, dans lequel l'élément de raccordement comprend un fil fait d'un corps isolant.

4. Dispositif médical (20) selon la revendication 1, dans lequel au moins un parmi le premier fil et le second fil comprend un fil multibrin comprenant une pluralité de fils d'élément métallique toronnés.

5. Dispositif médical (20) selon la revendication 4, dans lequel le premier fil comprend des fils d'élément métallique revêtus d'un corps isolant.

6. Dispositif médical (20) selon la revendication 1, dans lequel le second fil comprend un corps isolant.

7. Dispositif médical (20) selon la revendication 1, dans lequel le fil de manipulation comprend un corps isolant.

8. Dispositif médical (20) selon l'une quelconque des revendications 1 à 7, dans lequel le dispositif médical comprend un outil de traitement (50) comprenant : une partie de réception d'énergie (59) disposée à la partie d'insertion de l'outil de traitement pour recevoir le champ magnétique CA ou le champ électrique CA ; et une partie de traitement (51) qui est disposée sur le côté extrémité avant pour réaliser un traitement avec de l'énergie reçue.
